# EUROPÄISCHE PATENTSCHRIFT

(11) **EP 3 206 676 B1**
(45) Veröffentlichungstag und Bekanntmachung des Hinweises auf die Patenterteilung: **21.12.2022**
(21) Anmeldenummer: 15791520.8
(22) Anmeldetag: 19.10.2015
(51) Int. Cl.: A61K 31/19, A61P 37/02

(54) **MITTEL ZUR UNTERSTÜTZENDEN IMMUNMODULATION BEI MULTIPLER SKLEROSE UND PSORIASIS ENTHALTEND PROPIONSÄURE**
AGENT FOR THE SUPPORTIVE IMMUNE MODULATION IN MULTIPLE SCLEROSIS AND PSORIASIS CONTAINING PROPIONIC ACID
AGENT D'IMMUNOMODULATION DE SOUTIEN DANS LA SCLÉROSE EN PLAQUES ET LE PSORIASIS CONTENANT DE L'ACIDE PROPIONIQUE

(30) Priorität: 17.10.2014 DE 102014015314
(43) Veröffentlichungstag der Anmeldung: 23.08.2017
(73) Patentinhaber: Flexopharm Brain GmbH & Co. KG, 44628 Herne (DE)
(72) Erfinder: GOLD, Ralf, 44799 Bochum (DE); HAGHIKIA, Aiden, 44789 Bochum (DE); LINKER, Ralf, 91080 Uttenreuth (DE)
(74) Vertreter: Schneiders & Behrendt Bochum
(86) Internationale Anmeldenummer: PCT/EP2015/074179
(87) Internationale Veröffentlichungsnummer: WO 2016/059254

(56) Entgegenhaltungen:
- WO-A1-2010/105112
- BERG JOHANNES ET AL: "Beneficial effects of short chain fatty acids on the course of experimental autoimmune encephalomyelitis", JOURNAL OF NEUROIMMUNOLOGY, Bd. 275, Nr. 1, 15. Oktober 2014 (2014-10-15), Seite 59, XP029083324, ISSN: 0165-5728, DOI: 10.1016/J.JNEUROIM.2014.08.154
- PATRICK M SMITH ET AL: "The Microbial Metabolites, Short-Chain Fatty Acids, Regulate Colonic Treg Cell Homeostasis", SCIENCE, Bd. 341, Nr. 6145, 2. August 2013 (2013-08-02), Seiten 569-573, XP055247108, US ISSN: 0036-8075, DOI: 10.1126/science.1237947
- YUKIHIRO FURUSAWA ET AL: "Commensal microbe-derived butyrate induces the differentiation of colonic regulatory T cells", NATURE, Bd. 504, Nr. 7480, 13. November 2013 (2013-11-13), Seiten 446-450, XP055178312, ISSN: 0028-0836, DOI: 10.1038/nature12721
- A Haghikia ET AL: "Impact of fatty acids on CNS autoimmunity and their therapeutic potential for multiple sclerosis", , 7. Oktober 2015 (2015-10-07), Seiten 2-2, XP055247643, Gefunden im Internet: URL:http://citenpl.internal.epo.org/wf/web /citenpl/citenpl.html [gefunden am 2016-02-04]
- DUSCHA ALEXANDER ET AL: "Propionic Acid Shapes the Multiple Sclerosis Disease Course by an Immunomodulatory Mechanism", CELL, ELSEVIER, AMSTERDAM, NL, vol. 180, no. 6, 10 March 2020 (2020-03-10), page 1067, XP086100512, ISSN: 0092-8674, DOI: 10.1016/J.CELL.2020.02.035 [retrieved on 2020-03-10]

## Beschreibung

Die Erfindung betrifft ein Mittel zur Verwendung in der Begleitung der Therapie von Multipler Sklerose und Psoriasis.

Während bei Autoimmunerkrankungen körpereigene Strukturen Ziel einer fehlgesteuerten Immunantwort sind, beispielsweise bei Multipler Sklerose MS, kommt es bei immunmediierten chronischen Entzündungserkrankungen zu Entzündungen verschiedener Gewebe, wie etwa am Darm (Morbus Crohn, Colitis ulcerosa), der Haut (Schuppenflechte) oder den Gelenken (rheumatischer Formenkreis). Allen genannten Krankheitszuständen ist gemeinsam, dass in der Folge der Entzündungen weitere Erkrankungen überdurchschnittlich häufig auftreten können, wie Übergewicht, Bluthochdruck, Arteriosklerose, Herzinfarkt und Schlaganfall.

Jüngste Erkenntnisse auf dem Gebiet der Mikrobiome haben gezeigt, dass Ernährung, das intestinale Mikrobiom und die lokale zelluläre Immunantwort miteinander verknüpft sind. Dies legt nahe, dass mit diätetischen Maßnahmen Einfluss auf die zelluläre Immunantwort und damit auf den Verlauf von Autoimmunerkrankungen sowie immunmediierten chronischen Entzündungserkrankungen genommen werden kann.

Eine zentrale Rolle spielen dabei regulatorische T-Zellen (Treg) sowie die Diversität des Mikrobioms. Trotz vieler offener Fragen, insbesondere welche Komponenten des Mikrobioms tatsächlich für eine differenzierte adaptive Immunantwort im Darm verantwortlich sind, ergeben sich zunehmend empirische Hinweise darauf, dass einzelne Bakterienarten und ihre bakteriellen Metabolite einen starken Einfluss auf die systemische Immunantwort Zusammenhang mit Erkrankungen und Autoimmunität und immunmediierten chronischen Entzündungserkrankungen haben, beispielsweise für den Diabetes Typ 1und den Morbus Crohn.

Es hat sich ferner gezeigt, dass das Mikrobiom des Darms durch die Art der Nahrung beeinflusst werden kann und in der Lage ist, sich an die Anforderungen der jeweiligen Nahrung anzupassen. Dies bedeutet, dass eine für den Immunstatus eines Patienten ungünstige Darmflora durch diätetische Maßnahmen dahingehend geändert werden kann, dass sich der Immunstatus des Patienten verbessert. DUSCHA Alexander und Co-Autoren haben festgestellt, dass in einem EAE-Modell Propionsäure die Symptomatik verbessern kann, wenn sie bei Induktion der Krankheit gegeben wird. Eine spätere Gabe bei bereits vorhandenen Symptomen blieb ohne Effekt. In Anbetracht der Produktion von SCFAs durch Darmbakterien wird eine Anpassung der Ernährung in Betracht gezogen ("Beneficial effects of short chain fatty acids on the course of experimental autoimmune encephalomyelitis", JOURNAL OF NEUROIMMUNOLOGY, Bd. 275, Nr. 1, 15. Oktober 2014, Seite 59)

Das Mikrobiom des Darms wie auch Ernährungsgewohnheiten, etwa hohe Salzaufnahme, wurden kürzlich als Umwelteinflüsse auf die Pathogenese von multipler Sklerose (MS) als Prototyp einer von T-Zellen vermittelten Autoimmunerkrankung des zentralen Nervensystems identifiziert. Der Einfluss des Darm-Mikrobioms auf chronisch entzündliche Darmerkrankungen und Diabetes Typ 1 wurde bereits erwähnt. Auch bei Patienten, die an Diabetes Typ 2 leiden, wurden Besonderheiten in der Darmflora festgestellt.

Einen besonderen Einfluss auf die regulatorischen T-Zellen und das Mikrobiom im Darm haben Fettsäuren. Es wurde gefunden, dass langkettige Fettsäuren einen hemmenden Einfluss sowohl auf die regulatorischen T-Zellen im Darm als auch auf die Darmflora aufweisen. Überraschend ist, dass kurzkettige Fettsäuren eine positive Wirkung auf die Vermehrung und damit die Zahl der regulatorischen T-Zellen im Darm sowie auch im Blut haben. Dies gilt insbesondere für Propionsäure, Buttersäure und deren physiologisch vertretbaren Salze und Ester. Es wurde ferner gefunden, dass die gezielte Verabreichung kurzkettiger Fettsäuren mit drei bis acht Kohlenstoffatomen einen positiven Einfluss auf das Entstehen und den Verlauf von neuroimmunologischen Erkrankungen mit neurodegenerativen Aspekten haben, wie beispielsweise MS.

Entsprechend betrifft die Erfindung ein Mittel der eingangs genannten Art, das Propionsäure, ihre physiologisch vertretbaren Salze und/oder Ester mit C₁₋C₈-Alkylalkoholen enthält.

Das erfindungsgemäße Mittel kann als Nahrungsergänzungsmittel mit immunmodulierender Wirkung eingesetzt werden, wie auch zur Herstellung eines Medikaments zur Begleitung der Therapie von Autoimmunerkrankungen und immunmediierten chronischen Entzündungserkrankungen sowie deren Folgeerkrankungen, nämlich MS und Psoriasis.

Als Autoimmunerkrankungen kommen in erster Linie solche infrage, deren Entstehung mit Auffälligkeiten in der Darmflora und in Vorkommen von regulatorischen T-Zellen verbunden sind, nämlich MS und Psoriasis.

Der erfindungsgemäße Effekt ist auf kurzkettige Carbonsäuren, nämlich Propionsäure beschränkt. Bei längerkettigen Carbonsäuren schlägt der Effekt ins Gegenteil um; Carbonsäuren mit zwölf oder mehr Kohlenstoffatomen zeigen in der Regel einen negativen Einfluss auf die Entwicklung und den Verlauf der Erkrankung.

Als physiologisch verträgliche Salze kommen in erster Linie die Alkali- und Erdalkalisalze in Frage, dazu Salze von physiologisch unbedenklichen oder essentiellen Schwermetallen, etwa Zink oder Eisen. Besonders bevorzugt sind bei den Alkalimetallen Natrium und Kalium und bei den Erdalkalisalzen Magnesium und Calcium.

Bei den Estern sind die Methyl- und Ethylester bevorzugt.

Die erfindungsgemäße Propionsäure und ihre Ester und Salze kann mit Fumarsäureestern und -salzen sowie mit Vitamin A und D kombiniert werden, beispielsweise mit Dimethylfumarat und Salzen von Fumarsäuremonomethylester.

Das erfindungsgemäße Mittel kann grundsätzlich in jeder marktfähigen Form vorliegen. Bevorzugt sind Kapseln und Tabletten. Soweit die flüssigen Säuren oder Ester eingesetzt werden, ist eine Kapsel angesagt. Die Säuren in fester Salzform, etwa als Natriumpropionat können auch mit üblichen Tablettierungshilfsmitteln zu Tabletten gepresst werden.

Kapseln und Tabletten enthalten in der Regel eine Einheitsdosis von 0,2 bis 5 g, insbesondere von 0,5 bis 3 g.

Die erfindungsgemäßen Mittel können in einer Tagesdosis von bis zu 10 g verabreicht werden. In der Regel reicht aber eine Kapsel oder Tablette mit maximal 5 g pro Tag aus.

Des Weiteren kann das Mittel als Nahrungsergänzungsmittel mit immunmodulierender Wirkung eingesetzt werden.

Das erfindungsgemäße Mittel hat Auswirkungen auf die Darmphysiologie und das dortige Mikrobiom. So nimmt es Einfluss auf die Zusammensetzung des Mikrobioms. Die Anzahl an Propionat abbauenden Bakterien erhöht sich signifikant; der Einfluss auf die normale Darmflora bleibt dagegen gering. Dem gegenüber vermindern langkettige Carbonsäuren (Laurinsäure) die Zahl der Provotellaceae und einiger Familien von Bacteroidetes phylum bei Mäusen signifikant.

In mit Propionsäure behandelten Mäusen nimmt mit der Veränderung der Flora auch die Zahl der regulatorischen T-Zellen zu (CD4⁺ CD25⁺ Foxp3⁺ Treg). Genexpressionsanalysen von Signaturzytokinen zeigen erhöhte Werte für TGFß1, IL-10 - im Allgemeinen anti-entzündliche Botenstoffe - und Foxp3 mit Propionsäure gefütterten Mäusen mit experimenteller autoimmuner Enzephalomyelitis (EAE).

Die aliphatische Kettenlänge der Carbonsäuren hat ferner eine Auswirkung auf die Th1/Th7-vermittelte Autoimmunität und die regulatorische Antwort der Treg im Mausmodell in vivo. Mäuse mit einer an Laurinsäure angereicherten Diät zeigten in EAE-Modell eine signifikante Reduktion der TH1- und TH17-Zellen im Dünndarm und gleichzeitig eine Ansammlung von Th1/Th17 im zentralen Nervensystem, was dafür spricht, dass die Steuerung der entzündlichen Zellen aus dem Darm ins Gehirn/Rückenmark gefördert wird. Propionsäure führte dagegen unter ansonsten gleichen Bedingungen zu einer deutlichen Zunahme von TGFß1, IL-10 und Foxp3. Insgesamt ergab sich im Modell der MS, die einer Diät mit langkettigen Fettsäuren unterworfen wurden, gegenüber einer Kontrollgruppe eine Verschlechterung des Zustands nach Einsetzen der induzierten Erkrankung, bei Mäusen die Propionsäure erhielten, bei prophylaktischer Anwendung, eine deutliche Verbesserung.

Im Ergebnis scheint Propionsäure in der Lage zu sein, ein gestörtes Gleichgewicht von Treg und Effektor-T-Zellen (Th1/Th17) zu ändern beziehungsweise zu normalisieren. Gerade MS-Patienten weisen ein solch gestörtes Gleichgewicht auf.

### Experimentelles

Unter standardisierten Bedingungen gehaltene Mäuse wurden normal, mit einer an langkettigen Fettsäuren (30,9%. Laurinsäure) angereicherten Diät und 200 µl täglich oral verabreichten Propionat ernährt. Das Propionat wurde entweder zum Zeitpunkt der Induktion der Erkrankung (DI) oder bei Eintritt der Erkrankung (OD) verabreicht.

Zur Induktion der EAE wurden die Mäuse anästhetisiert und mit insgesamt 200 µg MOG₃₅₋₅₅ und 200 µg Freund Adjuvans (CFA) mit 4 mg/ml M. Tuberkulosis in zwei Subkutaninjektionen von 50 µl Emulsion links und rechts der Schwanzbasis behandelt. Pertussistoxin (200 mg/Maus) wurde intraperitoneal an den Tagen 0 und 2 nach der Induktion verabreicht. Die klinische Bewertung wurde täglich nach einer 5 Punkte-Skala (SEM) vorgenommen. Die Bewertung war wie folgt:
0 = normal
1 = Schwanzlähmung, beeinträchtigt das Aufrichten
2 = Gangataxie
3 = Paraparese der Hinterbeine
4 = Tetraparese
5 = Tod

Mäuse mit einem SEM von 4 oder 5 wurden ausgeschlossen.

Die Ergebnisse sind in den Abbildungen dargestellt.

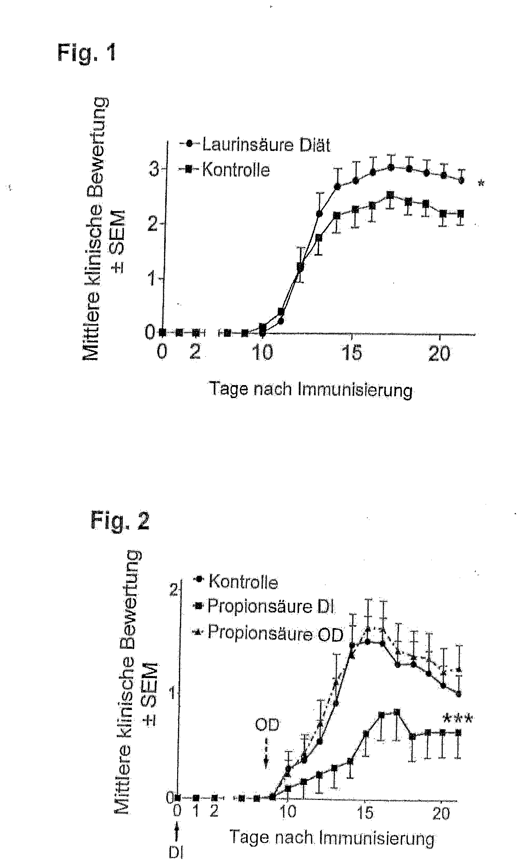

Abbildung 1 zeigt eine Auflistung der Mäusepopulation, die mit einer an Laurinsäure reichen Diät gefüttert wurden gegenüber einer Kontrollgruppe. Die Erkrankung setzte nach etwa zehn Tagen ein und erreichte nach siebzehn Tagen ihren Höhepunkt. Die Kontrollgruppe schnitt hinsichtlich der SEM-Werte besser ab als die Gruppe mit der an Laurinsäure reichen Diät.

Abbildung 2 zeigt ein Diagramm, in dem die mit Propionsäure gefütterte Mäusepopulation einer Kontrollgruppe gegenüber gestellt wurde. Dabei wurde die Propionsäure entweder am Tag der Immunisierung (DI) oder am Tag des Auftretens der Symptome (OD) verabreicht. Es zeigte sich, dass die Gruppe, der die Propionsäure am Tage der Erkrankung verabreicht wurde, einen signifikanten günstigeren Verlauf der Erkrankung aufwies, als die Kontrollgruppe.

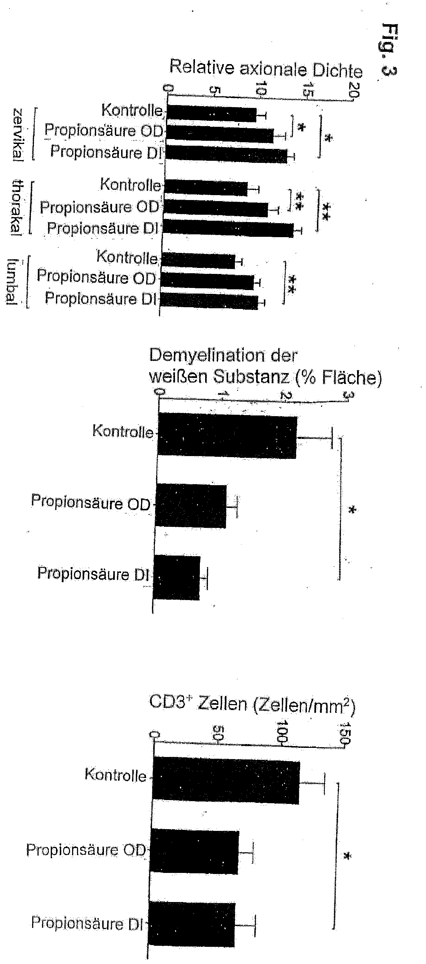

Abbildung 3 zeigt den Einfluss von Propionsäure auf die relative axionale Dichte, die Demyelination der weißen Substanz und die Zahl der CD3⁺⁻Zellen. In jedem Fall ergibt sich durch die Verabreichung von Propionsäure eine deutliche Verbesserung des Zustands gegenüber der Kontrollgruppe für Propionssäure.

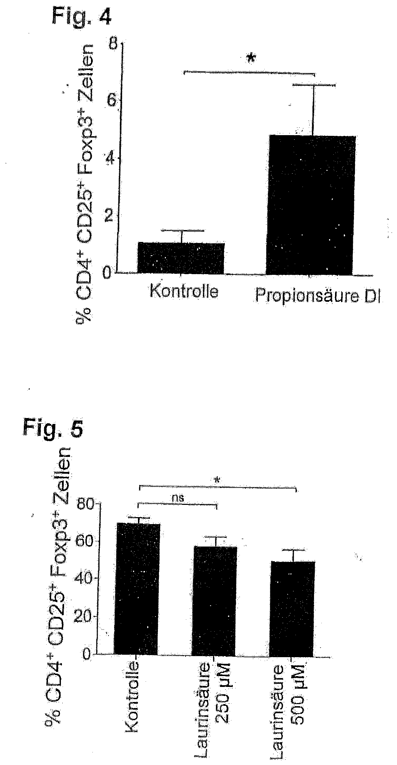

Abbildung 4 zeigt den Einfluss der Verabreichung von Propionsäure auf die CD4⁺⁻ CD25⁺ Foxp3-Zellen mit einer signifikanten Erhöhung gegenüber der Kontrollgruppe.

Abbildung 5 zeigt den Einfluss der an Laurinsäure reichen Diät auf die CD4⁺ CD25⁺ Foxp3-Zellen im Vergleich zu einer Kontrollgruppe. Die Verabreichung der langkettigen Fettsäure führt gegenüber dem Vergleichswert zu einer Verminderung der T-Zellen. Die Verminderung ist abhängig von der Konzentration der langkettigen Fettsäure.

## Patentansprüche

1. Mittel zur Verwendung in der Begleitung der Therapie von Multipler Sklerose und Psoriasis, enthaltend Propionsäure, ihre physiologisch vertretbaren Salze und/oder Ester mit C₁₋ C₈-Alkylalkoholen in einer Tagesdosis von bis zu 10g.

2. Mittel zur Verwendung bei der Behandlung von Multipler Sklerose und Psoriasis nach Anspruch 1, **dadurch gekennzeichnet, dass** die physiologisch vertretbaren Salze die Natrium, Kalium, Magnesium, Calcium, Zink und/oder Eisensalze sind.

3. Mittel zur Verwendung bei der Behandlung von Multipler Sklerose und Psoriasis nach einem der Ansprüche 1 oder 2, **dadurch gekennzeichnet, dass** es Natriumpropionat enthält.

4. Mittel zur Verwendung bei der Behandlung von Multipler Sklerose und Psoriasis nach einem der vorstehenden Ansprüche, **dadurch gekennzeichnet, dass** die physiologisch vertretbaren Ester die Methylester oder Ethylester sind.

5. Mittel zur Verwendung bei der Behandlung von Multipler Sklerose und Psoriasis nach einem der vorstehenden Ansprüche in Kapsel- oder Tablettenform.

6. Mittel zur Verwendung bei der Behandlung von Multipler Sklerose und Psoriasis nach Anspruch 5, **dadurch gekennzeichnet, dass** die Kapseln oder Tabletten eine Einheitsdosis von 0,2 bis 5 g, insbesondere 0,5 bis 3 g der Carbonsäuren, ihrer Salze oder Ester enthalten.

7. Nahrungsergänzungsmittel mit immunmodulierender Wirkung zur Verwendung in der Begleitung der Therapie von Multipler Sklerose und Psoriasis nach einem der Ansprüche 1 bis 6, enthaltend Propionsäure, ihre physiologisch vertretbaren Salze oder Ester mit C₁₋C₈-Alkylalkoholen, wie in einem der Ansprüche 1 bis 6 definiert.

## Claims

1. An agent for use alongside the therapy of multiple sclerosis and psoriasis, containing propionic acid, its physiologically tolerable salts and/or esters with C₁-C₈ alkyl alcohols in a daily dose of up to 10 g.

2. The agent for use in the treatment of multiple sclerosis and psoriasis according to Claim 1, **characterised in that** the physiologically tolerable salts are the sodium, potassium, magnesium, calcium, zinc and/or iron salts.

3. The agent for use in the treatment of multiple sclerosis and psoriasis according to either of Claims 1 or 2, **characterised in that** it contains sodium propionate.

4. The agent for use in the treatment of multiple sclerosis and psoriasis according to any of the preceding claims, **characterised in that** the physiologically tolerable esters are the methyl esters or ethyl esters.

5. The agent for use in the treatment of multiple sclerosis and psoriasis according to any of the preceding claims in capsule or tablet form.

6. The agent for use in the treatment of multiple sclerosis and psoriasis according to Claim 5, **characterised in that** the capsules or tablets contain a unit dose of 0.2 to 5 g, in particular 0.5 to 3 g of the carboxylic acids or the salts or esters thereof.

7. A nutritional supplement with an immunomodulatory effect for use alongside the therapy of multiple sclerosis and psoriasis according to any of Claims 1 to 6, containing propionic acid, its physiologically tolerable salts or esters with C₁-C₈ alkyl alcohols, as defined in any of Claims 1 to 6.

## Revendications

1. Agent destiné à être utilisé en accompagnement du traitement de la sclérose en plaques et du psoriasis contenant de l'acide propionique, ses sels et/ou esters physiologiquement acceptables avec des alcools d'alkyle en C₁-C₈ en une dose quotidienne allant jusqu'à 10 g.

2. Agent destiné à être utilisé lors du traitement de la sclérose en plaques et du psoriasis selon la revendication 1, **caractérisé en ce que** les sels physiologiquement acceptables sont les sels de sodium, potassium, magnésium, calcium, zinc et/ou de fer.

3. Agent destiné à être utilisé lors du traitement de la sclérose en plaques et du psoriasis selon l'une quelconque des revendications 1 ou 2, **caractérisé en ce qu'**il contient du propionate de sodium.

4. Agent destiné à être utilisé lors du traitement de la sclérose en plaques et du psoriasis selon l'une quelconque des revendications précédentes, **caractérisé en ce que** les esters physiologiquement acceptables sont les esters méthyliques ou les esters éthyliques.

5. Agent destiné à être utilisé lors du traitement de la sclérose en plaques et du psoriasis selon l'une quelconque des revendications précédentes sous forme de capsule ou de comprimé.

6. Agent destiné à être utilisé lors du traitement de la sclérose en plaques et du psoriasis selon la revendication 5, **caractérisé en ce que** les capsules ou les comprimés contiennent une dose unitaire de 0,2 à 5 g, en particulier de 0,5 à 3 g des acides carboxyliques, de leurs sels ou esters.

7. Complément alimentaire avec une action immunomodulatrice destiné à être utilisé en accompagnement du traitement de la sclérose en plaques et du psoriasis selon l'une quelconque des revendications 1 à 6, contenant de l'acide propionique, ses sels ou esters physiologiquement acceptables avec des alcools d'alkyle en C₁-C₈, tels que définis dans l'une quelconque des revendications 1 à 6.
